(19) Europäisches Patentamt

European Patent Office

Office européen des brevets

(11) **EP 4 175 692 B1**

(12) **EUROPEAN PATENT SPECIFICATION**

(45) Date of publication and mention
of the grant of the patent:
**20.08.2025 Bulletin 2025/34**

(21) Application number: **21745383.6**

(22) Date of filing: **05.07.2021**

(51) International Patent Classification (IPC):
***A61M 1/16*** (2006.01)

(52) Cooperative Patent Classification (CPC):
**A61M 1/1686; A61M 1/1688;** A61M 1/155

(86) International application number:
**PCT/GB2021/051705**

(87) International publication number:
**WO 2022/008889 (13.01.2022 Gazette 2022/02)**

(54) **DIALYSIS SYSTEM**

DIALYSESYSTEM

SYSTÈME DE DIALYSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.07.2020 GB 202010352**

(43) Date of publication of application:
**10.05.2023 Bulletin 2023/19**

(73) Proprietor: **Quanta Dialysis Technologies Limited
Warwick, Warwickshire CV34 5AH (GB)**

(72) Inventor: **WALLACE, Mark
Alcester Warwickshire B49 6EU (GB)**

(74) Representative: **Range, Christopher William
Withers & Rogers LLP
2 London Bridge
London SE1 9RA (GB)**

(56) References cited:
WO-A1-96/25214        DE-A1- 19 933 223
US-A1- 2015 273 090        US-A1- 2017 197 020

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Technical Field**

The present disclosure relates to a dialysis system. Particularly, but not exclusively, the disclosure relates to heat sanitization of a dialysis machine and a water purification system for use with a dialysis machine. Aspects of the present disclosure relate to dialysis system, and a method of heat sanitization of a dialysis water circuit.

**Background**

Dialysis is a treatment which replaces the renal function of removing excess fluid and waste products, such as potassium and urea, from blood. The treatment is either employed when renal function has deteriorated to an extent that uremic syndrome becomes a threat to the body's physiology (acute renal failure) or, when a longstanding renal condition impairs the performance of the kidneys (chronic renal failure).

There are two major types of dialysis, namely hemodialysis and peritoneal dialysis. In hemodialysis, the patient's blood is removed from the body by an arterial line and treated by a dialysis machine before being returned to the patient's body by a venous line. The machine passes the blood through a dialyser containing tubes formed from a semi-permeable membrane. On the exterior of the semi-permeable membrane is a dialysis fluid. The semi-permeable membrane filters the waste products and excess fluid from the blood into the dialysis fluid. The membrane allows the waste and a controlled volume of fluid to permeate into the dialysis fluid whilst preventing the loss of larger more desirable molecules, like blood cells and certain proteins and polypeptides.

The correction of uremic acidosis of the blood is achieved by use of a bicarbonate buffer. The bicarbonate buffer also allows the correction of the blood bicarbonate level. The dialysate fluid consists of a sterilized solution of mineral ions. These ions are contained within an acid buffer which is mixed with the purified water and bicarbonate base prior to delivery to the dialyser.

Production of dialysis fluid is described in the applicant's own applications, WO2010/146342, WO2010/146344, WO2014/155121 and WO2016/120415.

Further relevant prior art is for instance disclosed in documents DE19933223 A1, WO96/25214 A1 and US2015/273090 A1.

In simple terms, dialysis water is mixed with the bicarbonate buffer and the acid buffer to create a dialysis fluid for performing dialysis across a semi-permeable membrane in a dialyzer. Dialysis water is defined by the standard ISO 23500-3:2019. Dialysis fluid is defined by the standard ISO 23500-5:2019. The dialysis water may be generated by a water purification system which purifies domestic tap water by passing it through a prefiltration stage and then a reverse osmosis (RO) machine. Once supplied to the dialysis machine the dialysis water is heated to the correct temperature for treatment and is provided to the dialysis fluid mixing and pumping cassette where the acid and bicarbonate solutions are added to create dialysis fluid.

Patients being treated for a renal condition are typically required to either attend a medical facility, either in an acute setting, for example an intensive care ward or in a chronic setting, for example a dialysis ward or dialysis center. Some patient's requiring treatment for chronic conditions may be able to conduct dialysis at home.

Given the varied treatment settings, there is also a variation in the availability of dialysis water. For example a hospital dialysis ward may have access to a hospital water ring main, where dialysis water is provided from a hospital plant room. This may differ from an acute setting, such as an intensive care unit, where there is no provision of a hospital ring main. This may also differ from home use, where the only plentiful source of water is through the domestic tap. Given the varied treatment settings for dialysis, some may require the dialysis machine alone, whereas others may require both the dialysis machine and the RO machine.

Typically dialysis machines and RO machines need to be sanitized between uses and maintained in a sanitized condition. Conventionally, known dialysis machines are sanitized either through heat disinfection or chemical disinfection to ensure the microbiological quality of the supplied dialysis water and dialysis fluid is kept below acceptable levels as defined by the standard ISO 23500-5:2019. RO machines are sanitized through either through heat disinfection or chemical disinfection to ensure the microbiological quality of the supplied dialysis water is kept below acceptable levels as defined by the standard ISO 23500-3:2019. Water treatment equipment is governed by the standard ISO 23500-2:2019, and dialysis machines specifically by the standard IEC 60601-2-16:2019.

Such heat sanitization processes may be achieved by means of the A0 method which uses a knowledge of the lethality to microbiological contaminants of the particular process at different temperatures to assess the overall lethality to microbiological contaminants of the cycle and express this as the equivalent exposure time at a specified temperature. An example of the application of the A0 method to a dialysis machine is described in WO2015/185920.

Previous heat sanitizing systems for RO machines, such as the one disclosed in WO2018/202321, use a combination of a heating element located in the RO machine and chemical disinfectant in order to sanitize RO machines. This sanitization system only cleans the RO machine and not the dialysis machine attached to it.

RO machines and dialysis machines have independent sanitization routines. As such, the RO and dialysis machine must be sanitized independently. Often the devices must be disconnected from each other prior to the sanitization cycle. There is still the potential for bacteria to collect and to develop into biofilm in the fluid lines that connect the RO and dialysis machine as a result.

Known dialysis and RO systems are provided as independent systems with no shared functionality of control between the RO and dialysis machine when they are fluidly connected.

## Summary of the Invention

A portable dialysis system according to the present invention comprises the technical features as defined in independent claim 1. A method of heat sanitization of a portable dialysis system according to the present invention comprises the technical features as defined in independent claim 10.

In accordance with an aspect of the present invention, there is provided a portable dialysis machine having a main body portion; a water purification system, the water purification system being separate to the dialysis machine; and a liquid sanitizer; wherein the liquid sanitizer is provided within the main body portion of the dialysis machine; wherein the liquid sanitizer is fluidly connected between the dialysis machine and the water

purification system,
the liquid sanitizer having a heater arranged to heat a volume of liquid, a temperature sensor arranged to sense the temperature of the volume of liquid and a liquid sanitizer controller, the dialysis system defining a first closed fluid circuit comprising the dialysis machine and the liquid sanitizer and a second closed fluid circuit comprising the water purification system, the dialysis machine and the liquid sanitizer, and wherein the liquid sanitizer is configured to effect sanitization of the first closed fluid circuit and the second closed fluid circuit.

The liquid sanitizer heater is sized for heating dialysis water at 37 degrees Celsius from a typical inlet temperature of 10 degrees Celsius and at the typical flow rate of 500 ml/min in a dialysis treatment mode. This allows the dialysis machine to produce a continuous supply of dialysis fluid to be sent to a dialyser during treatment.

In liquid sanitization mode, the liquid sanitizer heater is required to heat dialysis water to approximately 80 to 85 degrees Celsius. Further, in liquid sanitization mode, the liquid sanitizer heater is required to maintain the heated dialysis water at approximately 80 to 85 degrees Celsius. In liquid sanitization mode a finite quantity of dialysis water is circulation around a closed fluid circuit of the dialysis machine at approximately 400 ml/min.

Since in liquid sanitization mode the sanitizing liquid is circulated around a closed fluid circuit and not used to prepare a continuous supply of dialysis fluid, the volume of dialysis water that must be heated is far smaller than in treatment mode and it is also increasing in temperature as it circulates around the closed fluid circuit. The liquid sanitizer heater therefore has spare capacity to effect further fluid heating.

The present invention takes advantage of that spare capacity to extend the closed fluid circuit to optionally further include the water purification system, and thereby increase the fluid volume of the closed fluid circuit. The present invention also takes advantage of that spare capacity to account for any additional heat loss experienced by circulating the heated fluid around the extended closed fluid circuit including the water purification system. The present invention further minimises the time between treatments which is necessary to sanitize the dialysis machine and the water purification system and the operator effort required therefore.

Since the liquid sanitizer is provided within the dialysis machine, it is not necessary for the water purification system to have a heater, thus the water purification system can be smaller, simpler, and inherently more reliable.

The liquid sanitizer controller may be controlled by a dialysis machine controller. The present invention allows for central control of the liquid sanitization of both the dialysis machine and the separate water purification system. This supports the ease of use of the dialysis system.

The dialysis machine may be provided with a graphical user interface, wherein the graphical user interface can provide instructions to the dialysis machine controller.

The water purification system may comprise a reverse osmosis machine.

The reverse osmosis machine may have a controller.

The liquid sanitizer controller may be configured to determine a time-temperature value for the volume of liquid periodically once a threshold temperature has been exceeded and calculate a cumulative time-temperature value for the first closed fluid circuit and the second closed fluid circuit.

The liquid sanitizer temperature sensor may be an inlet water temperature sensor arranged on a tank inlet adjacent a liquid sanitizer tank.

A liquid sanitizer outlet valve may be positioned adjacent a liquid sanitizer outlet to control the flow via either the liquid sanitizer outlet or a liquid sanitizer return line.

The RO machine controller may be controlled by the dialysis machine controller. Typically, whilst the dialysis machine is positioned at the optimum ergonomic height for the user, liquid sanitizers are often less accessible. Therefore control of the RO machine via the dialysis machine offers a better end-user experience.

In accordance with a further aspect of the present invention there is provided a method of heat sanitization of a portable dialysis system, the method comprising the steps of:

> providing a dialysis machine, a water purification system, and a liquid sanitizer,
> the liquid sanitizer being fluidly connected between the dialysis machine and the water purification system,
> the liquid sanitizer having a heater arranged to heat a volume of liquid and a temperature sensor arranged to sense the temperature of the volume of liquid,
> heating the volume of liquid from an initial temperature to exceed a threshold temperature, maintaining the volume of water above the threshold temperature, and circulating the volume of liquid through a first closed fluid circuit comprising the dialysis machine and the liquid sanitizer and circulating the volume of liquid through a second closed fluid circuit comprising the water purification system, the dialysis machine and the liquid sanitizer to effect a sanitizing dose in the first fluid circuit and the second fluid circuit.

The steps of circulating the volume of liquid through the first closed fluid circuit and circulating the volume of liquid through the second closed fluid circuit may be terminated in a sequential fashion.

The method may comprise the further steps of determining a time-temperature value for the volume of liquid periodically once the threshold temperature has been exceeded and calculating a cumulative time-temperature value based upon the determined time-temperature value.

The cumulative time-temperature value may be calculated according to

$$A_0 = \sum 10 e^{\frac{T-80}{z}} dt$$

A0 is the A value when z is 10°C;
t is the chosen time interval, in seconds;
and is the temperature in the load in °C.

The A0 value for the first closed fluid circuit may be equal to $A0_1$ and the A0 value for the second closed fluid circuit may be equal to $A0_2$, where $A0_2$ is greater than $A0_1$.

The step of circulating the volume of liquid through a second closed fluid circuit may include using a water purification system pump.

The method may comprise the further step of providing an output signal once the cumulative time-temperature value has reached a level indicative of a sanitizing dose in both the first fluid circuit and the second closed fluid circuit.

The method may comprise the further step of ceasing circulation through the second closed fluid circuit once the cumulative time-temperature value equals a target cumulative time-temperature value.

The method may comprise the further step of setting at least one of the threshold temperature, the upper temperature or the target cumulative time-temperature value.

## Brief description of the Drawings

One or more of the embodiments of the invention can now be described, by way of example only, with reference to the accompanying drawings, in which:

> Figure 1 is a schematic view of a system comprising of a reverse osmosis machine connected to a dialysis machine; and

> Figure 2 is a schematic of the system shown in Figure 1.

## Detailed Description

A dialysis system liquid sanitization system 10 comprises a dialysis machine 100, a water purification system and a liquid sanitizer 200 fluidly connected there between. The water purification system is separate to the dialysis machine 100. In the specific example, the water purification system comprises a reverse osmosis (RO) machine 300.

The dialysis machine 100 has a main body 112 with a door 114 hinged to a forward-facing side of the main body 112. The door 114 has a graphical user interface. The graphical user interface is a Liquid Crystal Display (LCD) unit 116 disposed on an outward-facing door surface and a door platen disposed on an inward-facing surface. The LCD unit 116 is touch sensitive allowing user inputs to control the dialysis machine 100 and liquid sanitization system 10 as will be described in more detail below. In alternative embodiments user input can be provided using buttons, dials or other suitable pieces of apparatus.

The door 114 closes against the main body 112 to define a recess 118 there-between. A dialysis fluid mixing and pumping cartridge 130 may be housed in the recess 118 as disclosed in WO2006120415, WO2015022537 and WO2018/178651.

The main body 112 has a platen 120 behind which is an engine portion (not shown for clarity). The platen 120 is configured to receive the dialysis fluid mixing and pumping cartridge 130 within the recess 118. The engine portion includes a pneumatic pump for providing pressure and vacuum to operate the dialysis machine 100.

The dialysis machine 100 is further provided with a dialysis machine controller 150.

The liquid sanitizer 200 is provided within the dialysis machine 100. The liquid sanitizer 200 is provided within the main body 112 of the dialysis machine 100. A dialysis machine fluid circuit 140 is fluidly connected to the liquid sanitizer 200 as will be described in more detail below.

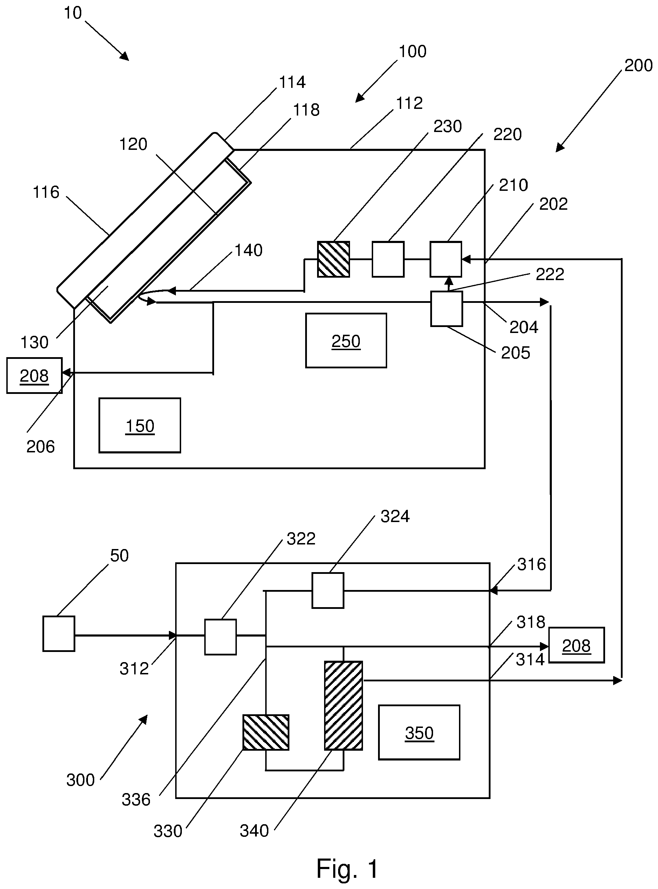

The dialysis machine has a network of fluid pathways generally designated fluid circuit 140. In dialysis use - the dialysis treatment mode - the dialysis machine fluid circuit 140 is connectable to the dialysis fluid mixing and pumping cartridge 130, where dialysis water heated to approximately 37 degrees Celsius is drawn off. In sanitization use - liquid sanitization mode - the dialysis machine fluid circuit 140 is not connected to the dialysis fluid mixing and pumping cartridge 130, instead dialysis water heated to approximately 80 to 85 degrees Celsius is not drawn off to the dialysis fluid mixing and pumping cartridge 130 but follows the arrows on Figure 1 along the dialysis machine fluid circuit 140.

The liquid sanitizer 200 has a number of fluid connections comprising a liquid sanitizer inlet 202, a liquid sanitizer outlet 204, a liquid sanitizer drain port 206 and a liquid sanitizer return line 222. The liquid sanitizer drain port 206 is fluidly connected to a drain 208.

The liquid sanitizer 200 has an inlet water temperature sensor 210, a liquid sanitizer tank 220, a liquid sanitizer pump 230, and a liquid sanitizer controller 250. The liquid sanitizer pump 230 may be a de-aeration pump, or another form of suitable pump. The liquid sanitizer pump 230 is disposed downstream of the liquid sanitizer tank 220 and inlet water temperature sensor 210. The liquid sanitizer tank 220 has a liquid sanitizer heater 240.

The liquid sanitizer inlet 202 is fluidly connected to the liquid sanitizer tank 220 with the inlet water temperature sensor 210 adjacent the liquid sanitizer inlet 202, upstream of the liquid sanitizer tank 220. The liquid sanitizer pump 230 is fluidly connected to the liquid sanitizer tank 220. The dialysis machine fluid circuit 140 is fluidly connected to the liquid sanitizer pump 230. The dialysis machine fluid circuit 140 is fluidly connected to the liquid sanitizer outlet 204 and separately, to the inlet water temperature sensor 210 via a liquid sanitizer return line 222. A liquid sanitizer outlet valve 205 positioned adjacent the liquid sanitizer outlet 204 controls the flow via either the liquid sanitizer outlet 204 or the liquid sanitizer return line 222.

Thus within the liquid sanitization system 10 a closed fluid circuit is provided comprising the inlet water temperature sensor 210, the liquid sanitizer tank 220, the liquid sanitizer pump 230, the dialysis machine fluid circuit 140 and the liquid sanitizer return line 222. This is the first closed fluid circuit.

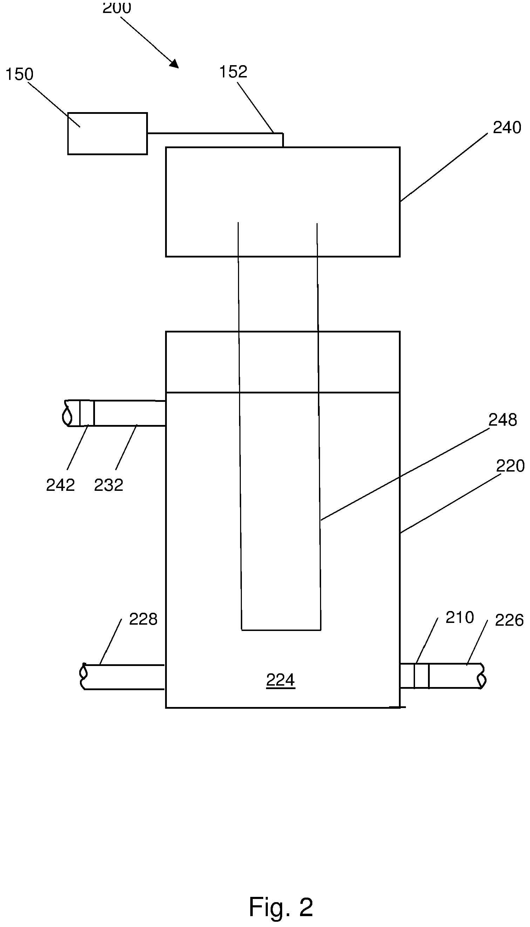

The detailed structure of the liquid sanitizer 200 is shown in Figure 2. The liquid sanitizer tank 220 contains, in use, a volume of water 224. The liquid sanitizer tank 220 has a tank inlet 226, a tank drain 228 and a tank outlet 232. The tank inlet 226 is fluidly connectable to a water source (the reverse osmosis machine). The tank drain 228 is fluidly connectable to the drain 208. The tank outlet 232 is fluidly connected to the liquid sanitizer pump 230.

The liquid sanitizer heater 240 has a heating element 248 arranged to heat the volume of water 224 contained within the liquid sanitizer tank 220, in this case by immersion in the volume of water 224. The liquid sanitizer heater 240 is electronically connected to the dialysis machine controller 150 by a heater connector 152.

Temperature sensors are arranged in the liquid sanitizer 200. An outlet temperature sensor 242 is arranged on the tank outlet 232 adjacent the liquid sanitizer tank 220. The inlet water temperature sensor 210 is arranged on the tank inlet 226 adjacent the liquid sanitizer tank 220. The temperature sensors 242, 210 are electronically connected to the dialysis machine controller 150 via sensor connectors. The connectors may be wired or wireless. The dialysis machine controller 150 may be remote to both the liquid sanitizer tank 220 and liquid sanitizer heater 240. The dialysis machine controller 150 thereby controls both the heating of the water and receives the temperature values for the sanitizing water circuit.

Referring back to Figure 1, the reverse osmosis (RO) machine 300 has an RO pump 330, an RO filter membrane 340 and an RO controller 350.

The RO controller 350 is connected to the dialysis machine controller 150. The dialysis machine controller 150 can communicate with and control the RO controller 350.

The reverse osmosis (RO) machine 300 has a number of fluid connections comprising an RO inlet 312, an RO outlet 314, an RO return 316, an RO drain 318. The RO inlet 312 is connected to a water source 50, such as a domestic tap, via a prefiltration stage.

The RO inlet 312 is fluidly connected to the RO pump 330 with an RO non-return valve (NRV) 322 adjacent the RO inlet 312. A common fluid line 336 fluidly connects the RO inlet 312 to the RO pump 330 downstream of the RO inlet NRV 322. The RO pump 330 is fluidly connected to the RO filter membrane 340. The RO filter membrane 340 is fluidly connected to the RO outlet 314, and separately to the RO drain.

The RO return 316 is fluidly connected to the RO pump 330, with a RO return NRV 324 adjacent the RO return 316. The common fluid line 336 fluidly connects the RO return 316 to the RO pump 330 downstream of the RO return NRV 324.

The RO drain 318 is fluidly connected to the common fluid line 336, downstream of both the RO inlet NRV 322 and RO return NRV 324.

Liquid Sanitization System

The RO machine 300 is fluidly connected to a dialysis machine 100 and liquid sanitiser 200 as shown in Figure 1. The RO outlet 314 is fluidly connected to the liquid sanitizer inlet 202. The RO return 316 is fluidly connected to the liquid sanitizer outlet 204.

The fluid connections are made by fluid lines shown schematically in Figure 1. The fluid lines may be made of medical grade plastic or other suitable material. The fluid lines may be provided with thermally insulating covers which prevent thermal losses due to heat conduction.

Thus within the liquid sanitization system 10 a further closed circuit is provided comprising the inlet water temperature sensor 210, the liquid sanitizer tank 220, the liquid sanitizer pump 230, the dialysis machine fluid circuit 140, the RO return 316, the RO return NRV 324, the RO pump 330, the RO filter membrane 340 and the RO outlet 314. This is the second closed fluid circuit.

General Setup

Depending upon the treatment setup, the dialysis machine 100 may be setup alone, or as part of a dialysis system liquid sanitization system 10 comprising the dialysis machine 100 and the water purification system. A portable unit may be used, as described in GB2006488.7 .

The water purification system is removably provided within the portable unit, whereas the dialysis machine 100 is removably provided on an upper surface of the portable unit.

Dialysis Treatment Mode

The dialysis machine 100 is used in a treatment mode to perform dialysis on a patient in a treatment session. The dialysis fluid mixing and pumping cartridge 130 is used to mix dialysis fluid constituent parts together with dialysis water from the RO machine 300, and supply the mixed dialysis fluid to a dialyser in specific quantities at specific flow rates. The RO machine 300 in the treatment mode is operable to pump water at high pressure using the RO pump 330 (between 5 and 40 bar) across the RO filter membrane 340. This generates purified water and waste water. The purified water is pumped to the dialysis machine 100 via the RO outlet 314. The waste water is pumped to the drain 208 via the RO drain 318. The ratio of generated purified water and waste water is known as the RO recovery rate. Typically, 50% of the supplied water results in dialysis water and 50% of the supplied water results in waste water.

Liquid Sanitization Mode

In between treatment sessions, it is necessary to sanitize the fluid connections of the dialysis machine 100 and the RO machine 300. This can be done to the dialysis machine 100 individually or to the dialysis machine 100 and the RO machine 300 combined at the same time.

The user turns on liquid sanitization system 10 via the graphical user interface of the dialysis machine 100. The appropriate Liquid Sanitization Mode is selected and thus the appropriate fluid circuit is made available.

To select the first closed fluid circuit, liquid sanitizer outlet valve 205 diverts dialysis water along the liquid sanitizer return line 222. To select the second first closed fluid circuit, liquid sanitizer outlet valve 205 diverts dialysis water out of liquid sanitizer outlet 204.

The appropriate fluid circuit is primed with dialysis water from the RO machine 300, which includes filling the liquid sanitizer tank 220 of the liquid sanitizer 200.

For the first closed fluid circuit the liquid sanitizer pump 230 is activated to pump the dialysis water around the first closed fluid circuit. The dialysis water is gradually heated from a typical initial temperature of 10 degrees Celsius to a target temperature of 80 to 85 degrees Celsius as it passes through the liquid sanitizer tank 220.

For the second closed fluid circuit the liquid sanitizer pump 230 and the RO pump 330 are activated to pump the dialysis water around the second closed fluid circuit. The RO machine controller 350 is controlled by the dialysis machine controller 150 in order to activate the RO pump 330. The dialysis water is gradually heated from a typical initial temperature of 10 degrees Celsius to a target temperature of 80 to 85 degrees Celsius as it passes through the liquid sanitizer tank 220.

Thus in both cases, the dialysis machine controller 150 activates the liquid sanitizer heater 240 to heat the volume of water 224 passing through the liquid sanitizer tank 220 via the heating element 248.

When sanitizing the dialysis machine only (first closed fluid circuit), the heated water is circulated around from the liquid sanitizer tank 220, the liquid sanitizer pump 230, the dialysis machine fluid circuit 140, the liquid sanitizer return line 222 and back to the liquid sanitizer tank 220 past inlet water temperature sensor 210.

When sanitizing the dialysis machine and the reverse osmosis machine (second closed fluid circuit), the heated water is circulated around from the liquid sanitizer tank 220, the liquid sanitizer pump 230, the dialysis machine fluid circuit 140, the RO return 316, the RO return NRV 324, the RO pump 330, the RO filter membrane 340 and the RO outlet 314 and back to the liquid sanitizer tank 220 past inlet water temperature sensor 210.

Again, in both cases, the dialysis water passes the inlet water temperature sensor 210 before re-entering the liquid sanitizer tank 220 as the dialysis water circulates around either the first or second closed fluid circuit.

The temperature of the water exiting the liquid sanitizer tank 220 via tank outlet 232 is periodically sensed by outlet temperature sensor 242, and the temperature data is periodically sent to liquid sanitizer controller 250. The temperature of the water returning to the liquid sanitizer tank 220 via tank inlet 226 is periodically sensed by inlet water temperature sensor 210, and the temperature data is periodically sent to liquid sanitizer controller 250. The liquid sanitizer controller 250 therefore periodically receives sensed temperature data to provide a feedback loop to moderate the heating of the volume of water 224 to maintain the temperature of the volume of water 224 above a threshold temperature. The threshold temperature is typically between 55 degrees Celsius and 65 degrees Celsius. The liquid sanitizer controller 250 may also moderate the heating of the volume of water 224 to maintain the temperature of the volume of water 224 below an upper temperature. The upper temperature may be between 85 degrees Celsius and 99 degrees Celsius.

When the liquid sanitizer controller 250 receives data from the inlet water temperature sensor 210 that the volume of water 224 has exceeded the threshold temperature, the liquid sanitizer controller 250 periodically samples the temperature of the volume of water 224 via the inlet water temperature sensor 210, which theoretically represents the lowest possible temperature of the water on either of first and second closed fluid circuits.

The sampling is performed periodically at, for example, 1 second intervals. The sampling intervals may be varied as appropriate. Each sampled temperature represents a time-temperature value, which can be calculated by the liquid sanitizer controller 250. The liquid sanitizer controller 250 calculates a cumulative time-temperature value for the volume of water 224 by summing the sampled time-temperature values. This is compared to a target total time-temperature value indicative of a sanitizing dose for either the first and second closed fluid circuits as appropriate.

Once the calculated cumulative time-temperature value and the target cumulative time-temperature value are equal, the liquid sanitizer controller 250 sends an output signal to indicate that a sanitizing dose has been reached. The output signal is received by the liquid sanitizer heater 240 and automatically switches off the liquid sanitizer heater 240. The output signal is received by the liquid sanitizer pump 230 which is automatically switched off. In the case of the second closed fluid circuit, the output signal is also received by the RO controller 350 which relays the signal to the RO pump 330 which is automatically switched off.

In an alternate embodiment, the liquid sanitizer controller 250 may switch off the liquid sanitizer heater 240 in advance of a sanitizing dose being reached, by calculating that there is sufficient thermal energy contained within the appropriate closed fluid circuit that the water temperature will remain above the threshold temperature for long enough to ensure a sanitizing dose is reached. In that case, periodic sampling would be continued, such that the liquid sanitizer controller 250 is able to send the output signal to indicate that a sanitizing dose had indeed been reached.

The output signal is received by the graphical user interface, which displays the text "COMPLETE" in reference to the completed sanitizing dose. In alternate embodiments, the graphical user interface includes an audible alarm. The audible alarm can be configured to bleep repeatedly until the liquid sanitization system 10 is turned off.

In alternate embodiments the liquid sanitizer controller 250 calculates two cumulative time-temperature values and two target total time-temperature value indicative of a sanitizing dose. A first cumulative time-temperature value for the first closed fluid circuit and a first target time-temperature value indicative of a sanitizing dose in the first closed fluid circuit. A second cumulative time-temperature value for the second closed fluid circuit and a second target time-temperature value indicative of a sanitizing dose in the second closed fluid circuit. Having two separate cumulative time-temperature values and target time-temperature values allows for optimised control of the sanitization of the RO and dialysis machine. The sanitization can be tailored to each component using the different sanitization target times.

The heat sanitization processes may be achieved by means of the A0 method which uses a knowledge of the lethality to biofilms of the particular process at different temperatures to assess the overall lethality to biofilms of the cycle and express this as the equivalent exposure time at a specified temperature.

The A value is a measure of the heat resistance of a microorganism. A is defined as the equivalent time in seconds at 80°C to give a sanitization effect. The z value indicates the temperature sensitivity of the reaction. It is defined as the change in temperature required to change the A value by a factor of 10. When the z value is 10°C, the term A0 is used. The A0 value of moist heat sanitization process is the equivalent time in seconds at a temperature of 80°C delivered by that process to the product with reference to microorganisms possessing a z value of 10°C.

$$A_0 = \sum 10 e^{\frac{T-80}{z}} dt$$

A0 is the A value when z is 10°C;
t is the chosen time interval, in seconds;
and is the temperature in the load in °C.

In calculating A0 values a temperature threshold for the integration is set at 65 °C since for temperatures below 65 °C the z and D value of thermophilic organisms may change dramatically and below 55°C there are a number or organisms which will actively replicate. In dialysis current practice, raising the temperature to 80°C for 30 minutes gives a benchmark value A0 equal to 1800.

The A0 value for the first fluid circuit comprising the dialysis machine is equal to $A0_1$. The A0 value for the second fluid circuit comprising the water purification system is equal to $A0_2$. $A0_2$ is greater than $A0_1$.

List of Reference Numerals

liquid sanitization system 10

water source 50
dialysis machine 100
main body 112
door 114
Liquid Crystal Display (LCD) unit 116
recess 118
platen 120
dialysis fluid mixing and pumping cartridge 130
dialysis machine fluid circuit 140
dialysis machine controller 150
heater connector 152
liquid sanitizer 200
liquid sanitizer inlet 202
liquid sanitizer outlet 204
liquid sanitizer outlet valve 205
liquid sanitizer drain port 206
drain 208
inlet water temperature sensor 210
liquid sanitizer tank 220
liquid sanitizer return line 222
volume of water 224
tank inlet 226
tank drain 228
liquid sanitizer pump 230
tank outlet 232
liquid sanitizer heater 240
outlet temperature sensor 242
heating element 248
liquid sanitizer controller 250
reverse osmosis (RO) machine 300
RO inlet 312
RO outlet 314
RO return 316
RO drain 318
RO non-return valve (NRV) 322
RO return NRV 324
RO pump 330
common fluid line 336
RO filter membrane 340
RO controller 350

**Claims**

1. A portable dialysis system (10) comprising:

   a dialysis machine (100) having a main body portion (112);
   a water purification system, the water purification system being separate to the dialysis machine (100);
   and a liquid sanitizer (200);
   wherein the liquid sanitizer (200) is provided within the main body portion (112) of the dialysis machine (100);
   wherein the liquid sanitizer (200) is fluidly connected between the dialysis machine (100) and the water purification system,

   the liquid sanitizer (200) having a heater (240) arranged to heat a volume of liquid, a temperature sensor (210, 242) arranged to sense the temperature of the volume of liquid and a liquid sanitizer controller (250),
   the dialysis system defining a first closed fluid circuit comprising the dialysis machine (100) and the liquid sanitizer (200), wherein the first closed fluid circuit is wholly within the dialysis machine main body portion (112), and a second closed fluid circuit comprising the water purification system, the dialysis machine (100) and the liquid sanitizer (200); and
   wherein the liquid sanitizer (200) is configured to effect sanitization of the first closed fluid circuit and the second closed fluid circuit.

2. The portable dialysis system (10) of claim 1, further comprising:
   a portable unit.

3. The portable dialysis system of claim 2, wherein the water purification system is removably provided within the portable unit, and the dialysis machine (100) is removably provided on an upper surface of the portable unit.

4. The portable dialysis system (10) of claim 1 wherein the liquid sanitizer controller (250) is controlled by a dialysis machine controller (150).

5. The portable dialysis system (10) of claim 2 wherein the dialysis machine (100) is provided with a graphical user interface, wherein the graphical user interface can provide instructions to the dialysis machine controller (150).

6. The portable dialysis system (10) of any preceding claim wherein the water purification system comprises a reverse osmosis machine (300), preferably wherein the reverse osmosis machine (300) has a controller (350), preferably still wherein the reverse osmosis machine controller (350) is controlled by the dialysis machine controller (150).

7. The portable dialysis system (10) of any preceding claim wherein the liquid sanitizer controller (250) is configured to determine a time-temperature value for the volume of liquid periodically once a threshold temperature has been exceeded and calculate a cumulative time-temperature value for the first closed fluid circuit and the second closed fluid circuit.

8. The portable dialysis system (10) of any preceding claim wherein the liquid sanitizer temperature sensor is an inlet water temperature sensor (210) arranged on a tank inlet adjacent a liquid sanitizer tank (220).

**9.** The portable dialysis system (10) of any preceding claim wherein a liquid sanitizer outlet valve (205) is positioned adjacent a liquid sanitizer outlet (204) to control the flow via either the liquid sanitizer outlet (204) or a liquid sanitizer return line (222).

**10.** A method of heat sanitization of a portable dialysis system (10), the method comprising the steps of:

providing a portable dialysis system (10) comprising a dialysis machine (100) having a main body portion (112), a water purification system, the water purification system being separate to the dialysis machine (100), and a liquid sanitizer (200), wherein the liquid sanitizer (200) is provided within the main body portion (112) of the dialysis machine (100);
the liquid sanitizer (200) being fluidly connected between the dialysis machine (100) and the water purification system,
the liquid sanitizer (200) having a heater (240) arranged to heat a volume of liquid and a temperature sensor (210, 242) arranged to sense the temperature of the volume of liquid,
heating the volume of liquid from an initial temperature to exceed a threshold temperature, maintaining the volume of water above the threshold temperature, and circulating the volume of liquid through a first closed fluid circuit comprising the dialysis machine (100) and the liquid sanitizer (200); and
circulating the volume of liquid through a second closed fluid circuit comprising the water purification system, the dialysis machine (100) and the liquid sanitizer (200) to effect a sanitizing dose in the first fluid circuit and the second fluid circuit.

**11.** The method according to claim 10, wherein the steps of circulating the volume of liquid through the first closed fluid circuit and circulating the volume of liquid through the second closed fluid circuit are terminated in a sequential fashion.

**12.** The method according to any of claims 10 or 11, comprising the further steps of: determining a time-temperature value for the volume of liquid periodically once the threshold temperature has been exceeded and calculating a cumulative time-temperature value based upon the determined time-temperature value.

**13.** The method according to claim 12, wherein the cumulative time-temperature value is calculated according to

$$A_0 = \sum 10 e^{\frac{T-80}{z}} dt$$

A0 is the A value when z is 10°C;
t is the chosen time interval, in seconds;
and is the temperature in the load in °C.

**14.** The method according to claim 13 where the A0 value for the first closed fluid circuit is equal to $A0_1$ and the A0 value for the second closed fluid circuit is equal to $A0_2$, where $A0_2$ is greater than $A0_1$.

**15.** The method according to any of claims 10 to 14 wherein the step of circulating the volume of liquid through a second closed fluid circuit includes using a water purification system pump.

**16.** The method according to any of claims 12 to 15 comprising the step of providing an output signal once the cumulative time-temperature value has reached a level indicative of a sanitizing dose in both the first fluid circuit and the second closed fluid circuit.

**17.** The method according to any of claims 12 to 15 comprising the step of ceasing circulation through the second closed fluid circuit once the cumulative time-temperature value equals a target cumulative time-temperature value.

**18.** The method according to claim 17 comprising the step of setting at least one of the threshold temperature, the upper temperature or the target cumulative time-temperature value.

**Patentansprüche**

**1.** Tragbares Dialysesystem (10), umfassend:

eine Dialysemaschine (100), die einen Hauptkörperabschnitt (112) aufweist;
ein Wasserreinigungssystem, wobei das Wasserreinigungssystem von der Dialysemaschine (100) getrennt ist;
und ein flüssiges Desinfektionsmittel (200);
wobei das flüssige Desinfektionsmittel (200) im Hauptkörperabschnitt (112) der Dialysemaschine (100) bereitgestellt ist;
wobei das flüssige Desinfektionsmittel (200) fluidisch zwischen der Dialysemaschine (100) und dem Wasserreinigungssystem verbunden ist,
wobei das flüssige Desinfektionsmittel (200) eine Heizung (240), angeordnet zum Erhitzen eines Flüssigkeitsvolumens, einen Temperatursensor (210, 242), angeordnet zum Erfassen der Temperatur des Flüssigkeitsvolumens, und eine flüssige Desinfektionsmittelsteuereinheit (250) aufweist,
wobei das Dialysesystem einen ersten geschlossenen Fluidkreislauf definiert, der die Dialysemaschine (100) und das flüssige Desinfektionsmittel (200) umfasst, wobei sich der erste geschlossene Fluidkreislauf vollständig innerhalb des Hauptkörperabschnitts (112) der Dialysemaschine befindet, und ein zweiter geschlossener Fluidkreislauf das Wasserreinigungssystem, die Dialysemaschine (100) und das flüssige Desinfektionsmittel (200) umfasst; und

wobei das flüssige Desinfektionsmittel (200) dazu konfiguriert ist, eine Desinfektion des ersten geschlossenen Fluidkreislaufs und des zweiten geschlossenen Fluidkreislaufs zu bewirken.

2. Tagbares Dialysesystem (10) nach Anspruch 1, weiter umfassend: eine tragbare Einheit.

3. Tragbares Dialysesystem nach Anspruch 2, wobei das Wasserreinigungssystem abnehmbar innerhalb der tragbaren Einheit bereitgestellt ist, und die Dialysemaschine (100) abnehmbar auf einer oberen Oberfläche der tragbaren Einheit bereitgestellt ist.

4. Tragbares Dialysesystem (10) nach Anspruch 1, wobei die flüssige Desinfektionsmittelsteuereinheit (250) durch eine Dialysemaschinesteuereinheit (150) gesteuert wird.

5. Tragbares Dialysesystem (10) nach Anspruch 2, wobei die Dialysemaschine (100) mit einer grafischen Benutzeroberfläche bereitgestellt ist, wobei die grafische Benutzeroberfläche Anweisungen an die Dialysemaschinensteuereinheit (150) bereitstellen kann.

6. Tragbares Dialysesystem (10) nach einem der vorstehenden Ansprüche, wobei das Wasserreinigungssystem eine Umkehrosmosemaschnine (300) umfasst, vorzugsweise wobei die Umkehrosmosemaschine (300) eine Steuereinheit (350) aufweist, vorzugsweise wobei die Steuereinheit (350) der Umkehrosmosemaschine durch die Steuereinheit (150) der Dialysemaschine gesteuert wird.

7. Tragbares Dialysesystem (10) nach einem der vorstehenden Ansprüche, wobei die flüssige Desinfektionssteuereinheit (250) so konfiguriert ist, dass sie periodisch einen Zeit-Temperatur-Wert für das Flüssigkeitsvolumen bestimmt, sobald eine Schwellentemperatur überschritten wurde, und einen kumulativen Zeit-Temperatur-Wert für den ersten geschlossenen Fluidkreislauf und den zweiten geschlossenen Fluidkreislauf berechnet.

8. Tragbares Dialysesystem (10) nach einem der vorstehenden Ansprüche, wobei der Temperatursensor für das flüssige Desinfektionsmittel ein Einlasswassertemperatursensor (210) ist, der an einem Tankeinlass neben einem Tank (220) für das flüssige Desinfektionsmittel angeordnet ist.

9. Tragbares Dialysesystem (10) nach einem der vorstehenden Ansprüche, wobei ein Auslassventil (205) für flüssiges Desinfektionsmittel neben einem Auslass (204) für flüssiges Desinfektionsmittel angeordnet ist, um den Durchfluss entweder über den Auslass (204) für flüssiges Desinfektionsmittel oder eine Rücklaufleitung (222) für flüssiges Desinfektionsmittel zu steuern.

10. Verfahren zur Hitzedesinfektion eines tragbaren Dialysesystems (10), wobei das Verfahren die Schritte umfasst:

Bereitstellen eines tragbaren Dialysesystems (10), umfassend eine Dialysemaschine (100), die einen Hauptkörperabschnitt (112), ein Wasserreinigungssystem, wobei das Wasserreinigungssystem von der Dialysemaschine (100) getrennt ist, und ein flüssiges Desinfektionsmittel (200), wobei das flüssige Desinfektionsmittel (200) im Hauptkörperabschnitt (112) der Dialysemaschine (100) bereitgestellt ist;

wobei das flüssige Desinfektionsmittel (200) fluidisch zwischen der Dialysemaschine (100) und dem Wasserreinigungssystem verbunden ist,

wobei das flüssige Desinfektionsmittel (200) eine Heizung (240) zum Erhitzen eines Flüssigkeitsvolumens und einen Temperatursensor (210, 242) aufweist, angeordnet zum Erfassen der Temperatur des Flüssigkeitsvolumens,

Erhitzen des Flüssigkeitsvolumens von einer Anfangstemperatur, um eine Schwellentemperatur zu überschreiten, Halten des Wasservolumens über der Schwellentemperatur, und Zirkulieren des Flüssigkeitsvolumens durch einen ersten geschlossenen Fluidkreislauf, der die Dialysemaschine (100) und das flüssige Desinfektionsmittel (200) umfasst; und

Zirkulieren des Flüssigkeitsvolumens durch einen zweiten geschlossenen Fluidkreislauf, der das Wasserreinigungssystem, die Dialysemaschine (100) und das flüssige Desinfektionsmittel (200) umfasst, um eine Desinfektionsdosis im ersten Fluidkreislauf und im zweiten Fluidkreislauf zu bewirken.

11. Verfahren nach Anspruch 10, wobei die Schritte des Zirkulierens des Flüssigkeitsvolumens durch den ersten geschlossenen Fluidkreislauf und des Zirkulierens des Flüssigkeitsvolumens durch den zweiten geschlossenen Fluidkreislauf nacheinander beendet werden.

**12.** Verfahren nach einem der Ansprüche 10 oder 11, umfassend die weiteren Schritte: periodisches Bestimmen eines Zeit-Temperatur-Werts für das Flüssigkeitsvolumen, sobald die Schwellentemperatur überschritten wurde, und Berechnen eines kumulativen Zeit-Temperatur-Werts basierend auf dem bestimmten Zeit-Temperatur-Wert.

**13.** Verfahren nach Anspruch 12, wobei der kumulative Zeit-Temperatur-Wert so berechnet wird, dass

$$A_0 = \sum 10 e^{\frac{T-30}{z}} dt$$

A0 der A-Wert ist, wenn z 10 °C beträgt;
t das gewählte Zeitintervall in Sekunden ist;
und die Temperatur in der Ladung in °C ist.

**14.** Verfahren nach Anspruch 13, wobei der A0-Wert für den ersten geschlossenen Fluidkreislauf gleich $A0_1$ ist und der A0-Wert für den zweiten geschlossenen Fluidkreislauf gleich $A0_2$ ist, wobei $A0_2$ größer ist als $A0_1$.

**15.** Verfahren nach einem der Ansprüche 10 bis 14, wobei der Schritt des Zirkulierens des Flüssigkeitsvolumens durch einen zweiten geschlossenen Fluidkreislauf eine Verwendung einer Wasserreinigungssystempumpe beinhaltet.

**16.** Verfahren nach einem der Ansprüche 12 bis 15, umfassend den Schritt des Bereitstellens eines Ausgangssignals, sobald der kumulative Zeit-Temperatur-Wert einen Pegel erreicht hat, der sowohl im ersten Fluidkreislauf als auch im zweiten geschlossenen Fluidkreislauf eine Desinfektionsdosis anzeigt.

**17.** Verfahren nach einem der Ansprüche 12 bis 15, umfassend den Schritt des Beendens der Zirkulation durch den zweiten geschlossenen Fluidkreislauf, sobald der kumulative Zeit-Temperatur-Wert einem kumulativen Zeit-Temperatur-Zielwert entspricht.

**18.** Verfahren nach Anspruch 17, umfassend den Schritt des Festlegens von mindestens einem von der Schwellentemperatur, der oberen Temperatur oder des kumulativen Zeit-Temperatur-Zielwerts.

**Revendications**

**1.** Système (10) de dialyse portable comprenant :

une machine (100) de dialyse ayant une partie (112) de corps principal ;
un système de purification d'eau, le système de purification d'eau étant séparé de la machine (100) de dialyse ;
et un désinfectant (200) liquide ;
dans lequel le désinfectant (200) liquide est prévu à l'intérieur de la partie (112) de corps principal de la machine (100) de dialyse ;
dans lequel le désinfectant (200) liquide est relié fluidiquement entre la machine (100) de dialyse et le système de purification d'eau,
le désinfectant (200) liquide ayant un réchauffeur (240) agencé pour chauffer un volume de liquide, un capteur (210, 242) de température agencé pour détecter la température du volume de liquide et un dispositif de commande (250) de désinfectant liquide,
le système de dialyse définissant un premier circuit de fluide fermé comprenant la machine (100) de dialyse et le désinfectant (200) liquide, dans lequel le premier circuit de fluide fermé est entièrement à l'intérieur de la partie (112) de corps principal de machine de dialyse, et un deuxième circuit de fluide fermé comprenant le système de purification d'eau, la machine (100) de dialyse et le désinfectant (200) liquide ; et
dans lequel le désinfectant (200) liquide est configuré pour effectuer une désinfection du premier circuit de fluide fermé et du deuxième circuit de fluide fermé.

**2.** Système (10) de dialyse portable selon la revendication 1, comprenant en outre :
une unité portable.

**3.** Système de dialyse portable selon la revendication 2, dans lequel le système de purification d'eau est prévu de manière amovible à l'intérieur de l'unité portable, et la machine (100) de dialyse est prévue de manière amovible sur une surface supérieure de l'unité portable.

**4.** Système (10) de dialyse portable selon la revendication 1 dans lequel le dispositif de commande (250) de désinfectant liquide est commandé par un dispositif de commande (150) de machine de dialyse.

**5.** Système (10) de dialyse portable selon la revendication 2 dans lequel la machine (100) de dialyse est dotée d'une interface utilisateur graphique, dans lequel l'interface utilisateur graphique peut fournir des instructions au dispositif de commande (150) de machine de dialyse.

**6.** Système (10) de dialyse portable selon une quelconque revendication précédente, dans lequel le système de purification d'eau comprend une machine (300) à osmose inverse, de préférence dans lequel la machine (300) à osmose inverse a un dispositif de commande (350), de préférence encore dans lequel le dispositif de commande (350) de machine à osmose inverse est commandé par le dispositif de commande (150) de machine de dialyse.

7. Système (10) de dialyse portable selon une quelconque revendication précédente, dans lequel le dispositif de commande (250) de désinfectant liquide est configuré pour déterminer périodiquement une valeur de temps-température pour le volume de liquide une fois qu'une température seuil a été dépassée et calculer une valeur de temps-température cumulée pour le premier circuit de fluide fermé et le deuxième circuit de fluide fermé.

8. Système (10) de dialyse portable selon une quelconque revendication précédente, dans lequel le capteur de température de désinfectant liquide est un capteur (210) de température d'eau d'entrée agencé sur une entrée de réservoir adjacente à un réservoir (220) de désinfectant liquide.

9. Système (10) de dialyse portable selon une quelconque revendication précédente, dans lequel une vanne (205) de sortie de désinfectant liquide est positionnée adjacente à une sortie (204) de désinfectant liquide pour contrôler le débit via soit la sortie (204) de désinfectant liquide, soit une conduite (222) de retour de désinfectant liquide.

10. Procédé de désinfection thermique d'un système (10) de dialyse portable, le procédé comprenant les étapes de :

   fourniture d'un système (10) de dialyse portable comprenant
   une machine (100) de dialyse ayant une partie (112) de corps principal, un système de purification d'eau, le système de purification d'eau étant séparé de la machine (100) de dialyse, et un désinfectant (200) liquide, dans lequel le désinfectant (200) liquide est prévu à l'intérieur de la partie (112) de corps principal de la machine (100) de dialyse ;
   le désinfectant (200) liquide étant relié fluidiquement entre la machine (100) de dialyse et le système de purification d'eau,
   le désinfectant (200) liquide ayant un réchauffeur (240) agencé pour chauffer un volume de liquide et un capteur (210, 242) de température agencé pour détecter la température du volume de liquide,
   le chauffage du volume de liquide à partir d'une température initiale pour dépasser une température seuil, le maintien du volume d'eau au-dessus de la température seuil, et la circulation du volume de liquide à travers un premier circuit de fluide fermé comprenant la machine (100) de dialyse et le désinfectant (200) liquide ; et
la circulation du volume de liquide à travers un deuxième circuit de fluide fermé comprenant le système de purification d'eau, la machine (100) de dialyse et le désinfectant (200) liquide pour effectuer une dose de désinfection dans le premier circuit de fluide et le deuxième circuit de fluide.

11. Procédé selon la revendication 10, dans lequel les étapes de circulation du volume de liquide à travers le premier circuit de fluide fermé et de circulation du volume de liquide à travers le deuxième circuit de fluide fermé sont terminées de manière séquentielle.

12. Procédé selon l'une quelconque de la revendication 10 ou la revendication 11, comprenant les étapes supplémentaires de : détermination d'une valeur temps-température pour le volume de liquide périodiquement une fois que la température seuil a été dépassée et calcul d'une valeur temps-température cumulée sur la base de la valeur temps-température déterminée.

13. Procédé selon la revendication 12, dans lequel la valeur temps-température cumulée est calculée selon

$$A_0 = \sum 10 e^{\frac{T-80}{z}} dt$$

   A0 est la valeur A lorsque z est de 10 °C ;
   t est l'intervalle de temps choisi, en secondes ;
   et est la température dans la charge en °C.

14. Procédé selon la revendication 13 où la valeur A0 pour le premier circuit de fluide fermé est égale à $A0_1$ et la valeur A0 pour le deuxième circuit de fluide fermé est égale à $A0_2$, où $A0_2$ est supérieur à $A0_1$.

15. Procédé selon l'une quelconque des revendications 10 à 14 dans lequel l'étape de circulation du volume de liquide à travers un deuxième circuit de fluide fermé inclut l'utilisation d'une pompe de système de purification d'eau.

16. Procédé selon l'une quelconque des revendications 12 à 15 comprenant l'étape de fourniture d'un signal de sortie une fois que la valeur temps-température cumulée a atteint un niveau indicatif d'une dose de désinfection à la fois dans le premier circuit de fluide et le deuxième circuit de fluide fermé.

17. Procédé selon l'une quelconque des revendications 12 à 15 comprenant l'étape d'arrêt de circulation à travers le deuxième circuit de fluide fermé une fois que la valeur temps-température cumulée est égale à une valeur temps-température cumulée cible.

**18.** Procédé selon la revendication 17 comprenant l'étape de réglage d'au moins l'une de la température seuil, de la température supérieure ou de la valeur temps-température cumulée cible.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010146342 A
- WO 2010146344 A
- WO 2014155121 A
- WO 2016120415 A
- DE 19933223 A1
- WO 9625214 A1
- US 2015273090 A1
- WO 2015185920 A
- WO 2018202321 A
- WO 2006120415 A
- WO 2015022537 A
- WO 2018178651 A
- GB 2006488 A